Europäisches Patentamt

European Patent Office

Office européen des brevets

(19)

(11) Publication number: **0 171 381**
A2

# EUROPEAN PATENT APPLICATION

(21) Application number: **85870105.5**

(22) Date of filing: **01.08.85**

(51) Int. Cl.⁴: **C 12 N 15/00**, A 01 N 63/00, C 12 N 1/20, C 12 N 5/00 // C12N9/24, C12N9/40 ,(C12N1/20, C12R1:38)

(30) Priority: **02.08.84 US 636888**
**02.07.85 US 750118**

(43) Date of publication of application: **12.02.86** Bulletin 86/7

(84) Designated Contracting States: **AT BE CH DE FR GB IT LI LU NL SE**

(71) Applicant: **Monsanto Company, Patent Department 800 North Lindbergh Boulevard, St. Louis Missouri 63167 (US)**

(72) Inventor: **Jaworski, Ernest George, 11 Clerbrook, St. Louis Missouri 63124 (US)**
Inventor: **Hemming, Bruce Clark, 804 Paquerette Court, Manchester Missouri 63011 (US)**
Inventor: **McPherson, Sylvia Ann, 2301 Divot Drive, St. Louis Missouri 63131 (US)**
Inventor: **Drahos, David Joseph, 13320 Hobnail Drive, St. Louis Missouri 63146 (US)**
Inventor: **Lawson, Edgar Clifford, 608 McKinley Avenue, St. Louis Missouri 63122 (US)**
Inventor: **Jonsson, Colleen Beth, 9520 Plainfield Street, St. Louis Missouri 63119 (US)**
Inventor: **Fuchs, Roy Lee, 11 Courtway Place, Ballwin Missouri 63011 (US)**

(74) Representative: **Lunt, John Cooper et al, Monsanto Europe S.A. Patent Department Avenue de Tervuren 270-272 Letter Box No 1, B-1150 Brussels (BE)**

(54) **Nematode control using soil bacteria.**

(57) This invention relates to bacteria which are capable of proliferating in a environment which is infested with nematodes (such as pseudomonads which colonize the surfaces of plant roots) under conditions of microbial competition, and which inhibit nematodes. This invention also relates to a method of using such bacteria to inhibit nematodes.

This invention also involves the transformation of bacteria with foreign genes which express glycosidase enzymes (such as β-galactosidase) and/or chitinase, which can inhibit one or more types of nematodes.

Methods are disclosed herein for (1) selecting suitable bacteria which can colonize a target environment, such as the root surfaces of a particular type of plant; (2) transforming a selected bacteria with foreign genes which encode chitinase and/or glycosidase enzymes, preferably under the control of a strong promoter; and (3) determining whether the bacteria effectively inhibit a selected type of nematode in the target environment.

# NEMATODE CONTROL USING SOIL BACTERIA

## BACKGROUND OF THE INVENTION

This invention is in the field of biochemistry, and relates to the use of soil bacteria and genetic engineering to control nematodes.

### Nematodes

A wide variety of small parasitic worms are referred to as "nematodes". Numerous types of nematodes, such as root knot nematodes (Meloidogyne species) and cyst nematodes (Heterodera species) are plant parasites and cause substantial damage to crops and other plants around the world. Other types of nematodes such as hookworms and heartworms are human and animal parasites.

The physiology, life cycle, and behavior of nematodes are discussed in several references, such as Lee 1971 and Dropkin 1980 (note: a complete list of all references with full citations is contained after the examples). Briefly, when a plant nematode egg is laid, the embryo inside the egg is referred to as an L1 larva. Before hatching, the embryo molts and forms an L2 larva. The L2 larva usually emerges from the egg within about 2 weeks, although under certain conditions, some types of nematode eggs can remain viable and hatch after a period of several years. The L2 larva which emerges from the egg is often called a preparasitic larva. Using its hardened tubular stylus, the L2 larva penetrates the surface of a root. Most L2 larvae feed for about two weeks, then go through a series of three molts without feeding. An immature adult emerges from the final molting, matures and lays eggs. As used herein, "nematode" refers to a nematode at any stage in its life cycle.

"Ectoparasitic" nematodes such as dagger or ring nematodes remain outside of roots and use long styli to suck nutrients out of root cells. They can transmit viral infections to plants and cause especially serious problems to grape vines and fruit trees.

"Endoparasitic" nematodes enter the roots and migrate to feeding zones within the roots. Migratory endoparasites, such as lesion or lance nematodes, do not stay in a single location in a plant; they usually lay their eggs within root tissue. Sedentary endoparasites, such as root knot or cyst nematodes, infect a root at a single location, and usually lay their eggs outside of the root tissue.

Most types of nematodes have chemotactic sensory organs called "amphids," which help the nematodes locate sources of food. The head regions of Caenorhabditis elegans (a soil nematode which eats bacteria) have been reported to be glycosylated with residues of glucose, galactose, mannose and sialic acid; see, e.g., McClure 1981 and 1982. However, work reported by Spiegel 1982A and 1982B indicated that M. javanica (a plant nematode) did not have any galactose moieties.

Plant nematodes cause severe economic damage to crops, and a variety of efforts have been made to inhibit nematodes. As used herein, "inhibit" is used broadly to refer to any activity which prevents or retards the effects of nematodes on other organisms such as plants, or which reduces the number of nematodes or nematode eggs in an area of interest. For example, nematode inhibition may relate to the destruction of larval nematodes or nematode eggs, or to decreasing the motion or infectivity of nematodes by inflicting physiological damage upon the nematodes. Inhibition may involve effects such as the failure of larvae to emerge from the egg, or the emergence of premature larvae which cannot survive or reproduce.

In field or greenhouse tests, evaluations of nematodes and nematicides usually monitor (1) the number of nematodes or nematode eggs which can be gathered from a given quantity of soil or roots, or (2) the amount of damage inflicted upon plants in the field. In *in vitro* studies, various other assays are used, such as (1) chemotactic response (e.g., movement by nematodes toward or away from a diffusible point source of a chemical); (2) changes in larval motility (most viable larvae of most species move almost constantly under certain conditions, so lack of motion under such conditions usually is a reliable indicator of non-viability); (3) changes in infectivity, as measured by the number of galls, cysts or eggs that can be gathered from soil or roots; or (4) vital staining.

A variety of fungi (including <u>Meria conio-spora</u>, <u>Dactylaria candida</u>, and <u>D. oviparasitica</u>) and at least two types of bacteria (<u>Bacillus penetrans</u> and <u>Ps. denitrificans</u>) can kill and digest nematodes. In addition, other naturally occurring bacteria (including pseudomonads) are reported to contain and/or release chitinase (see Nagahata 1979 and Warnes 1984), collagenase (see, e.g., Rao 1977 and Carrick 1973), and other proteolytic enzymes (see, e.g., Ikeda 1978).

However, naturally occurring fungi and bacteria have very limited effectiveness in protecting plants by inhibiting nematodes. Numerous efforts to use fungi or bacteria to inhibit plant nematodes have resulted in, at best, short term (e.g., single-season) protection against only a narrow range of nematodes. At least four reasons for the lack of greater success have been identified.

First, for bacteria to be truly effective in inhibiting nematodes which invade plant roots, the bacteria must effectively colonize the surfaces of the plant roots, and proliferate in high numbers despite intense competition from thousands of other microbes. Some nematocidal bacteria can exist in soil, and some can exist on root surfaces, but most cannot exist in sufficiently high numbers to be effective.

Second, if the nematode population decreases after an area is inoculated with nematocidal microorganisms, the population of nematocidal bacteria subsequently dwindles due to competition from other bacteria. After the nematocidal population dwindles, nematodes reappear, and eventually a balance of nematodes and nematocidal bacteria develops. The nematodes in a balanced population usually cause substantial damage to susceptible crops.

Third, most nematocidal bacteria have only narrow host ranges. For example, B. penetrans, which is effective against root knot nematodes, is ineffective against cyst nematodes. Although used as a biocontrol agent, B. penetrans is considered to be an obligatory parasite which is very difficult to culture or sustain in the absence of nematode hosts. Therefore, it is very difficult to prepare sufficient quantities of B. penetrans cells or spores to inoculate a sizable area, and any inoculum is likely to contain viable, infective nematodes.

Fourth, some bacteria will kill nematodes only under certain conditions. For example, most nematophagus fungi create nematode traps only under conditions of food shortage.

## Chitin and Chitinase

Nematode eggs contain chitin, a polymeric form of N-acetyl glucosamine (NAG). Although some researchers believe that larval nematodes also contain chitin (particularly in their styli), other researchers disagree; chitin has never been reliably reported to exist in larval or adult nematodes. Chitin is known to exist in insects and fungi.

As used herein, the phrase "chitinous pest" includes any organism or bacteria which exerts pathogenic, parasitic, or other detrimental effects on plants or animals, and which contains chitin during any stage in its life cycle. Chitinous pests include nematodes, fungi, and insects.

It has been reported by Miller 1977 that several substances (including wheat germ lipase, papain, chitinase, and collagenase) can reduce the motility of one type of nematode, Tylenchorhynchus dubius. This report did not indicate the type, source, or purity of the chitinase or other substances used. The Applicants have determined that commercial sources of chitinase contain non-specific hydrolytic and proteolytic enzymes which could inhibit nematodes.

Several types of bacteria, including Serratia marcescens and Pseudomonas stutzeri, are known to express and release chitinase enzymes. As used herein, "release" of an enzyme by a cell includes active secretion of the enzyme by viable cells, as well as release of the enzyme by dead cells.

Chitinase genes in some species are believed to be expressed at low levels in the absence of induction. If chitin is encountered and degraded by released chitinase, the resulting products can include monomers, dimers, and oligomers of NAG. These chitin degradation products are soluble, and it has been hypothesized (see, e.g., Monreal 1969) that they may diffuse into the bacteria, where they may induce greater expression of the chitinase gene(s).

This inducing effect is believed by some to be related to reports that chitin, if applied to the soil, can reduce nematode populations and infections. See, e.g., Mankau 1969.

As used herein, a "constitutive" promoter refers to a gene promoter which promotes the expression of a substantial or large quantity of a polypeptide, in the absence of conditions or substances which are recognized as "inducers" by those skilled in the art. This does not require continuous expression of the gene during all stages in the life of the cell (e.g., most genes are not expressed during certain replicative stages). This includes promoters which can be repressed by certain substances in certain types of hosts, if such repressing substances are not present in the desired environment or in the transformed host cells. Natural chitinase promoters are regarded herein as "inducible" rather than constitutive; see Monreal 1969 and Mankau 1969.

A variety of different types of chitinase have been reported. One category, often called "exochitinases," usually removes monomers or possibly oligomers of NAG from an end of a polymeric chitin molecule. By comparison, "endochitinases" can cleave chitin polymers at the junction between any two NAG residues. A different but related class of enzymes, called "chitobiases," cleaves dimers and possibly oligomers of NAG into monomeric NAG. Each category contains a variety of different chitinases which have different levels of activity on different forms and sources of chitin; for example, chitinases expressed by Serratia marcescens genes may differ from chitinases expressed by other microorganisms. As used herein, "chitinase" includes exochitinases and endochitinases, but not chitobiases.

Glycosidase Enzymes and Fluorescent Pseudomonads

Proteins, cells, and animal organs which have saccharide (sugar) moieties attached to them are said to be "glycosylated." A "glycosidase" enzyme (also called a "glycolytic" enzyme) is an enzyme which is capable of cleaving one or more saccharide moieties from a molecule such as a disaccharide, a polysaccharide, or a glycoprotein.

A variety of glycosidase enzymes are known, including beta-galactosidase (B-gal), which can cleave a beta-galactose linkage (such as the linkage in lactose). The B-gal enzyme in E. coli is encoded by a gene called lacZ. The lacZ gene is part of the lac operon, which contains an inducible promoter/operator region, the lacZ coding sequence, a lacY coding sequence which encodes lactose permease, and a lacA coding sequence which encodes thiogalactosidase transacetylase. Because of its inducible promoter system and its use as a chromogenic marker gene, the lac operon and the B-gal enzyme have been widely studied and used; see, e.g., Miller 1980.

Pseudomonad bacteria are of interest to plant scientists for several reasons. They are relatively abundant in the soil, and strains which colonize the roots of any specific cultivar of a selected crop can be easily obtained by harvesting cells from the roots of that cultivar. In addition, certain soil pseudomonads are believed to exert growth-inhibiting effects on some detrimental microorganisms, including pathogenic fungi. By impeding detrimental microorganisms, some pseudomonads have been shown to help protect seeds and seedlings; see Canadian Patent 1,172,585 (Schroth, U. of California) Pseudomonads are also of scientific and industrial interest in various other fields of technology, including the biodegradation of agricultural and industrial wastes, and various E. coli - pseudomonad shuttle vectors are available, allowing for relatively simple genetic manipulation in a laboratory.

Several species of pseudomonads, including Ps. fluorescens, are fluorescent when grown on certain types of nutrient media. Fluorescent pseudomonads create molecules, most of which are referred to as "siderophores", which are fluorescent; when these molecules (and the bacteria which contain them) are excited by light at a wavelength of about 290-390 nm (in the ultraviolet range), they emit light at a different wavelength, usually about 398-498 nm (in the yellow-green range). Fluorescent pseudomonads therefore can be easily distinguished from non-fluorescent pseudomonads in a laboratory by observing them under ultraviolet light. See, e.g., Meyer 1978, Shelly 1980, and Schroth 1981.

Application 592,158 (commonly assigned to Monsanto) discloses that naturally existing fluorescent pseudomonads do not contain B-gal activity. Based on that discovery, a method was created for using B-gal genes as marker genes to help scientists monitor the proliferation of inoculated, genetically transformed fluorescent pseudomonads in environments such as greenhouses and open fields.

## Objects of the Invention

One object of this invention is to create method for effectively inhibiting nematodes, using bacteria. Another object is to provide a method of inserting foreign genes into one or more types of soil bacteria which can colonize the roots of a selected type of plant, to provide that particular type of plant with effective protection against nematodes. Another object is to create compositions which can be applied to soil and which contain bacteria which inhibit nematodes.

## SUMMARY OF THE INVENTION

This invention relates to bacteria which are capable of proliferating in an environment which is infested with nematodes (such as pseudomonads which colonize the surfaces of plant roots) under conditions of microbial competition, and which inhibit nematodes. This invention also relates to a method of using such bacteria to inhibit nematodes and other chitinous pests.

This invention also involves the transformation of bacteria with foreign genes which express glycosidase enzymes (such as beta-galactosidase) and/or chitinase, which can inhibit one or more types of nematodes.

Methods are disclosed herein for (1) selecting suitable bacteria which can colonize a target environment, such as the root surfaces of a particular type of plant; (2) transforming a selected bacteria with foreign genes which encode chitinase and/or glycosidase enzymes, preferably under the control of a strong promoter; and (3) determining whether the bacteria effectively inhibit a selected type of nematode in the target environment.

## BRIEF DESCRIPTION OF THE FIGURES

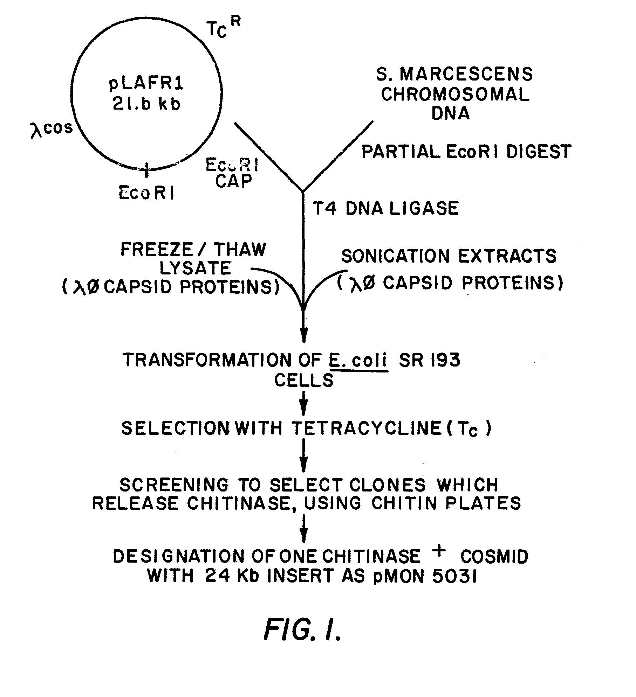

FIGURE 1 depicts the shotgun cloning of partially digested S. marcescens DNA into a cloning vector.

FIGURE 2 depicts the digestion of cosmid pMON5031 by EcoRI to create a 9.5 kb fragment, and the insertion of that fragment into pMON5012 to create plasmids pMON5035 and pMON5036.

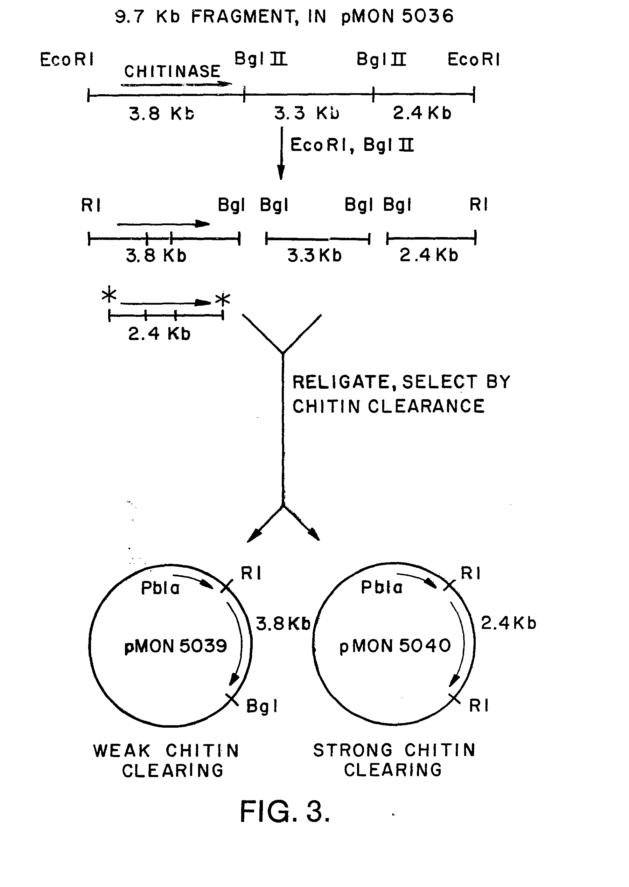

FIGURE 3 depicts the creation of pMON5039 with a 3.8 kb chitinase gene fragment, and pMON5040 with a 2.4 kb chitinase gene fragment.

FIGURE 4 depicts the chromatographic purification and separation of chitinases from S. marcescens.

FIGURE 5 depicts the nematode egg-hatching inhibiting activity of purified chitinase at concentrations ranging from 1 to 100 ug/ml.

DETAILED DESCRIPTION OF THE INVENTION

This invention relates to the use of bacteria which naturally exist in large numbers in symbiotic or non-detrimental relationships on plant roots, such as fluorescent pseudomonads, to inhibit nematodes.

Prior to this invention, there was no expectation that unmodified pseudomonads, when inoculated onto plant roots, would substantially inhibit nematodes. Untransformed pseudomonads were used as controls in genetic engineering experiments involving beta-galactosidase (B-gal) genes and chitinase genes. These experiments compared pseudomonads with foreign B-gal and/or chitinase genes against (1) pseudomonads with no foreign genes, or (2) soil with no inoculated bacteria. These experiments involved (1) unsterilized soil having a mixture of various naturally occurring bacteria; (2) soybean or tomato plants; (3) strains of P.s. fluorescens isolated from corn or soybean roots; and (4) root knot nematodes from the species Meloidogyne incognita or M. javonica.

The results, shown in Table 1, indicate that after 28 days, plant roots inoculated with pseudomonads having no foreign genes suffered from significantly fewer nematode galls compared to roots with no inoculated bacteria.

Statistical values are expressed as the mean value, plus or minus the "standard error of the mean" (s.e.m), calculated by the method described in Johnson 1980. In general, the mean value indicates the average of the data points, and the s.e.m. value indicates the distribution of the data around the mean value.

TABLE 1

EFFECTS OF UNTRANSFORMED PSEUDOMONADS

ON NEMATODES

| Pseudomonad strain | (Mean ± s.e.m) |
|---|---|
| Test No. 1 | |
| No inoculated bacteria | 53 ± 2.8 |
| 701E1 | 32.6 ± 3.5 |
| Test No. 2 | |
| No inoculated bacteria | 104.9 ± 10.7 |
| 112-12 | 58.6 ± 6.3 |

This demonstrates that pseudomonads can be inoculated onto plant roots to inhibit nematodes, without any genetic transformation.

In the use of pseudomonads (or other types of transformed bacteria as discussed below) to inhibit nematodes in a particular environment, it is important to select a strain of bacteria which can proliferate in that environment despite competition from naturally occurring microbes. Nearly any type of microbe which naturally proliferates in a specific environmental niche (such as on the roots of a particular type of plant, in a certain type of soil) must be well-adapted to survival in that niche. If such bacteria are inoculated into such an environment that is already colonized by a variety of other bacteria, there is a probability that the progeny of the inoculated bacteria would colonize the environment and exist in relatively large numbers after a period of time.

Microbes which can proliferate in any specific environment may be obtained simply by taking material (soil, root tissue, etc.) from that environment, and harvesting and culturing the microorganisms contained in or on such material. Alternately, the scientific literature abounds with references indicating types of microbes which naturally populate most types of environments that are of interest with regard to this invention. For example, it is known that soil bacteria from the genera Agrobacterium, Rhizobium, Azotobacter, Azospirillum, Flavobacterium, Acetobacter, Erwinia, Enterobacter, Klebsiella, Serratia, Citrobacter, Chromobacterium, Proteus, Gluconobacter, Corynebacterium, Streptomyces, Frankia, Nocardia, or Bacillus colonize various types of roots or exist in certain types of soil in high numbers.

Numerous strains of pseudomonads and other types of soil bacteria may be readily isolated from the roots of plants growing in various locations. In addition, numerous other strains are publicly available from depositories such as the American Type Culture Collection (Rockville, Maryland) and the Northern Regional Research Labs (Peoria, Illinois). Any deposited or isolated strain of bacteria (either transformed or untransformed) may be tested for effectiveness in inhibiting one or more types of nematodes on the roots of a particular type of plant in a specific type of soil, using the following procedures. The bacteria can be inoculated into one or more portions of a field which is infested with nematodes (or other pests), or into containers containing unsterilized soil from such a field. The bacteria can be inoculated onto the roots of plants, or onto seeds. Periodically, treated and untreated areas or containers can be assayed for nematode larva, egg, or cyst counts and for the presence of the inoculated bacteria, by the methods described herein or otherwise known to those skilled in the art. A reduction in the number of nematode counts in areas colonized by the bacteria, compared to areas not colonized, indicates that the bacteria inhibits the types of nematode(s) found in the untreated areas or containers.

Another aspect of this invention involves the transformation of bacteria by inserting foreign genes which cause the transformed cells and their progeny to express and release glycosidase enzymes. Such transformed cells have been shown to inhibit nematodes, as described below.

Cultures of two strains of <u>Ps. fluorescens</u>, designated as strains 701E1 and 1141, were transformed by inserting a chimeric lacZY gene (under the control of a strong constitutive promoter, the beta-lactamase or Pbla promoter) into their chromosomes. The resulting strains, designated as 701L5 and 1141L1, were deposited with the ATCC and were assigned accession numbers 53175 and 53176, respectively.

Originally, the B-gal genes were intended solely for use as marker genes to help count the inoculated bacteria, as described in U.S. application serial number 592,158. The B-gal enzyme was not expected to inhibit nematodes, and cells containing B-gal genes were used as controls in nematode inhibition experiments involving chitinase genes. However, it was discovered that the B-gal genes substantially increased the ability of the pseudomonads to inhibit nematodes, as shown in Table 2.

TABLE 2

EFFECTS OF B-GAL ENZYME

ON NEMATODES

| Inoculated strain | Gall Count (Mean ± s.e.m.) |
|---|---|
| None | 53 ± 2.8 |
| 1141L1 (with lacZY) | 31.8 ± 6.9 |
| 701E1 | 32.6 ± 3.5 |
| 701L1 (with lacZY) | 14.8 ± 3.7 |

Although the reasons for nematode inhibition by the B-gal enzyme is not yet fully understood, it is believed that certain glycosidase enzymes such as B-gal may be able to damage nematodes by attacking sugar moieties on or near the amphids. A program of evaluating the effects of other glycosidases on nematodes has been commenced. One such enzyme, mannosidase, has been shown to inhibit M. javanica nematodes in an in vitro test described in Example 1.C. Bacterial genes which encode mannosidase can be isolated using recombinant DNA technology, and inserted into soil bacteria to determine whether such transformed bacteria are capable of inhibiting nematodes. Similarly, genes which encode other enzymes which are shown to inhibit nematodes in vitro can be isolated and used to transform bacteria, which can then be tested on nematodes.

In addition, because of the enzymatic activity involved, it is believed that nematode inhibition by transformed bacteria does not depend upon the genus of the host cell involved, so long as the host cell can (1) express and release an active glycosidase enzyme from the inserted gene, and (2) colonize the target environment.

A third aspect of this invention involves the use of chitinase to inhibit nematodes. In an experiment described in Example 1.B, it was determined that purified chitinase from S. marcescens can inhibit the hatching of larvae from nematode eggs, at concentrations as low as 1 ug/ml. To determine whether this gene could be used to transform pseudomonads and cause them to inhibit nematodes, the chitinase gene from S. marcescens was isolated, placed in an expression vector, and used to transform pseudomonads, as described in the examples and briefly summarized below.

Chromosomal DNA from S. marcescens was randomly fragmented, and the DNA fragments were inserted into a cosmid cloning vector. The cosmid DNA's with inserts were packaged into lambda phage capsids. The resulting phages were used to insert the cosmid DNA into E. coli cells. Once inside the cells, the cosmid DNA functions as a plasmid rather than a phage, since it does not contain a full set of phage genes.

The transformed E. coli cells were spotted onto nutrient agar plates containing colloidal chitin overlays. The collodial chitin layer is translucent and insoluble. If chitinase which is capable of degrading the colloidal chitin is released by the transformed cells, the chitinase can degrade the colloidal chitin into soluble products, thereby creating a clear zone which is visible against the colloidal chitin background.

Six colonies surrounded by chitin clearance zones were selected and used for subsequent analysis. Each cosmid in a selected colony contained a segment of DNA from S. marcescens, approximately 22 to 26 kilobases (kb) long, which contained one or more chitinase genes.

Using a tri-parental mating, the modified cosmids from the 6 selected E. coli clones were transferred to Ps. fluorescens strain 701E1. Another screening step using chitin overlay plates was utilized, and the pseudomonad colony with the largest area of chitin clearing was selected. It contained a cosmid designated as pMON5031, which was deposited with the American Type Culture Collection (ATCC), accession number 39765.

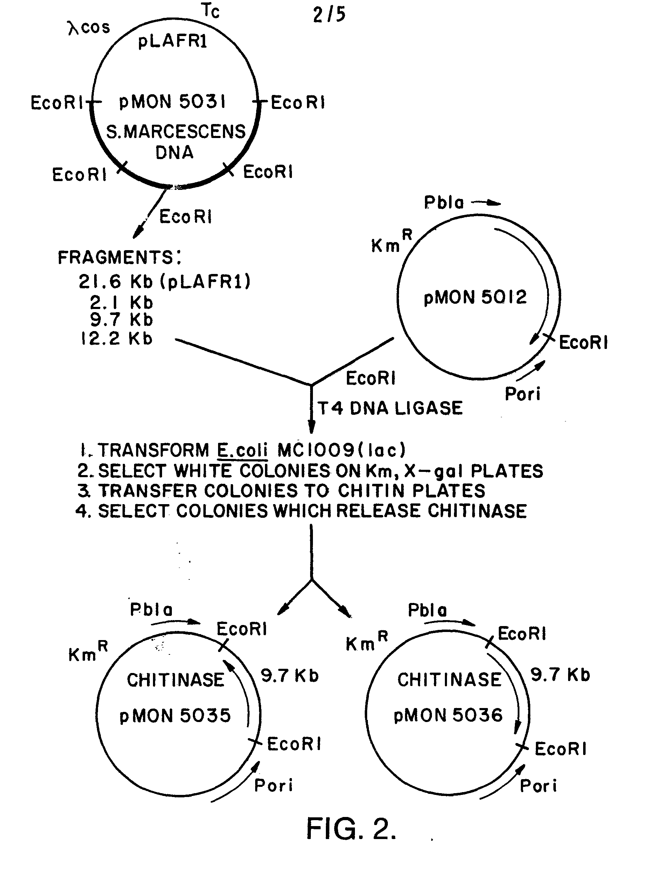

In order to increase the expression of the chitinase gene in both E. coli and pseudomonad cells, and to alleviate the dependence of expression upon possible induction by chitin, the 24 kb insert was removed from cosmid pMON5031 and digested into smaller fragments using various endonucleases, as described in Example 2.F. The resulting DNA fragments were inserted into a separate plasmid, and the resulting plasmids were transformed into E. coli cells. Colonies were selected and screened for chitinase release using chitin overlay plates. Two positive clonal colonies were identified, and the plasmids they contained were designated as pMON5035 and pMON5036. Each of those plasmids contains at least one chitinase gene on a 9.5 kb fragment bounded by EcoRI cleavage sites, having two internal BglII sites but no internal EcoRI sites.  In plasmid pMON5036, which causes a larger chitin clearing zone than pMON5035, the chitinase gene is apparently under the control of the Pbla promoter, as shown in Figure 2.  A culture of E. coli cells containing pMON5036 was deposited with the ATCC, and was assigned accession number 39764.

As described in Example 2.G, plasmid pMON5036 was removed from E. coli and digested with both EcoRI and BglII.  The resulting fragments were religated in order to create plasmids having subfragments of the 9.5 kb chitinase segment under the control of the Pbla promoter. The ligation mixture was transformed into E. coli cells, which were plated onto chitin overlay plates and screened for chitin clearing.

Five out of 200 clones tested had unusually large areas of chitin clearing.  Each of these clones contained a 2.4 kb insert; one was designated as pMON5040.  When these clones were analyzed by a variety of endonucleases, was discovered that the 2.4 kb segment was apparently created by EcoRI "star" cleavage (discussed in the 1983/84 New England Biolabs Catalog, at p. 77, and by Fuchs 1983) at a non-EcoRI site. The unexpected star cleavage apparently removed some form of transcription repressor from the chitinase gene on the 2.4 kb fragment, leading to higher levels of expression of the chitinase gene in pMON5040 than in previous vectors.  A culture of E. coli LE392 cells containing pMON5040 was deposited with the ATCC, and was assigned accession number 39774.

In a set of experiments described in Example 3, fluorescent pseudomonads containing (1) no foreign genes, (2) a B-gal gene, or (3) a B-gal gene and a chitinase gene, were inoculated into soil containing soybean seeds and root knot nematodes. This study lasted for 28 days (approximately 1 generation of nematodes). The results, shown in Table 3, indicated:

    (1)  the inoculated, untransformed bacteria
         (strain 701E1) inhibited nematodes
         compared to uninoculated controls when
         both were tested using unsterilized
         soil;

    (2)  bacteria containing the B-gal gene
         inhibited nematodes to a greater extent
         than comparable strains which did not
         contain B-gal genes; and,

    (3)  bacteria containing the chitinase gene
         inhibited nematodes to a greater
         extent than comparable strains which
         did not contain the chitinase gene.


TABLE 3

NEMATODE INHIBITION BY PSEUDOMONADS
WITH B-GAL AND CHITINASE GENES

| Bacterial Strain | Foreign Gene(s) | Gall Count (Mean ± s.e.m) |
|---|---|---|
| None (control) | - | 53 ± 2.8 |
| 1141L1 | LacZY | 31.8 ± 6.9 |
| 1141L1 (pMON5040) | LacZY, chitinase | 20.8 ± 2.6 |
| 701E1 | - | 32.6 ± 3.5 |
| 701L5 | LacZY | 14.8 ± 3.7 |
| 701L5(pMON5040) | LacZY, chitinase | 11.8 ± 1.9 |

Strains of bacteria which can colonize a target environment in high numbers despite microbial competition are good candidates for genetic transformation using the vectors and genes described herein, especially if they naturally inhibit nematodes to some degree before transformation. The glycosidase and/or chitinase genes of this invention can be used to transform any such bacteria, using methods and vectors described herein or otherwise known to those skilled in the art. Selection of optimal bacterial strains which inhibit nematodes most effectively in a selected environment can be determined using routine experimentation.

In addition, chitinase and/or glycosidase genes may be used to transform strains of enteric bacteria (such as certain Proteus or Serratia strains) which are used as feed supplements for some ruminant animals. Such transformed bacteria can be inoculated into the digestive systems of such animals, to inhibit certain types of nematodes such as Ascaris species which cause parasitic damage to animals.

This invention is not limited to chitinase genes from Serratia marcescens. Other bacteria, such as Serratia liquefaciens and Bacillus polymyxa, express various forms of chitinase that have different substrate activities and rates than chitinase expressed by S. marcescens. The chitinase genes from such microbes may be isolated, placed on expression vectors, and used to transform selected bacteria. If desired, a purified form of any type of chitinase may be tested in vitro to determine whether it inhibits one or more types of nematodes. Genes which encode chitinases that inhibit nematodes in vitro are good candidates for isolation and use as described herein.

If desired, the bacteria of this invention may also be transformed with chitobiase genes. This may allow the bacteria to cleave dimeric or oligomeric NAG (created by the digestion of chitin by chitinase) into monomeric NAG, which can be used as a nutrient by many types of bacteria. The isolation and cloning of a chitobiase gene was reported in Horwitz 1984.

As used herein, "inoculating" refers to any method of introducing viable bacteria into a selected environment. For example, root surfaces can be inoculated with bacteria by contacting the plant seeds with a liquid suspension of bacteria, drying the seeds, and planting the seeds. When the seeds sprout, the roots will be in contact with the bacteria.

If desired, bacteria which inhibit nematodes can be mixed with various types of carrier material, such as peat moss, vermiculite, soil, seeds or other plant tissue, methylcellulose, xanthan gum, etc. The mixture can be spread over, plowed into, or otherwise mixed with nematode-infested soil. If desired, the bacteria can be mixed with the material while in freeze-dried form, encapsulated in biodegradable or water-soluble material, or otherwise treated to prolong their viability or decrease their levels of metabolic activity during handling. If desired, the carrier material may contain assimilable nutrient sources to support proliferation of the bacteria. Bacteria intended for enteric inoculation can be mixed with carrier material that is suitable for ingestion by the intended animals.

If desired, the genes of this invention can be inserted into the chromosomes of bacteria, to ensure their stable inheritance. This can be useful if plasmids are unstable in a particular type of cell that is competing against other microbes in a specific environment. Various methods for inserting genes into the chromosomes of bacteria are known, such as the method described in EPO 091,723 (Grinter).

If desired, the DNA sequences which encode the selected enzyme(s) may be placed under the control of strong constitutive promoters, such as the beta-lactamase promoter (Pbla), the "tac" promoter, or bacteriophage promoters. The resulting chimeric genes will cause greater expression of the desired enzymes than the natural genes. Cultures of cells containing plasmids pMON5031, pMON5036, or pMON5040 (which contain chitinase genes) were deposited with the ATCC, and were assigned accession numbers 39765, 39764, and 39774, respectively. In addition, cultures of three Ps. fluorescens strains (701E1, which is rifampicin resistant but otherwise untransformed; 701L5, which is 701E1 with a chromosomal lacZY gene; and strain 1141L1, which is a different rifampicin resistant strain with a chromosomal lacZY gene) were deposited and assigned ATCC numbers 39773, 53175, and 53176, respectively.

Numerous types of variants and mutants of these cultures, or of the plasmids or genes therein, may be created by methods known to those skilled in the art. For example, the cells may be treated with mutagenic agents such as X-rays, ultraviolet light, or various chemicals which cause random mutations. Alternately, it is possible to alter single or multiple bases in a plasmid or gene in a controlled manner. Treated cells, or any host cell containing a treated plasmid or gene, may be assayed for nematode-inhibiting activity by the methods described above.

As used herein, a "bacteria containing a gene descended from" a deposited culture includes any bacteria which inherits or is transformed with DNA that is replicated from the DNA in a deposited culture. As one example, plasmid pMON5040 may be removed from culture #39774 (or its progeny) and inserted into a different microbial host. As another example, the chitinase gene may be removed from pMON5040, inserted into a vector that has different marker genes, and inserted into a selected host cell. Either type of transformed cell would be covered by the claims.

As used herein, a "genetically transformed

0171381

bacteria" includes all progeny or other descendants of a transformed bacteria. A "foreign" gene refers to a gene that is inserted into an existing cell, by transformation, mating, etc., and to such genes which are inherited by the progeny of the transformed cell. Foreign genes are often referred to as "heterologous" genes in the literature.

A "chimeric" gene refers to a functional gene created by ligating two or more gene fragments together. For example, a constitutive promoter ligated in proper relationship to a structural sequence (also called a coding sequence) which encodes chitinase is one type of chimeric gene. Chimeric genes could also be created by, for example, digesting a single gene into three fragments, removing one fragment which inhibits expression of the gene, and religating the remaining fragments together to reconstitute a modified gene with higher expression levels.

The following examples further describe certain specific embodiments of this invention. Those skilled in the art will recognize, or may ascertain using routine experimentation, numerous equivalents to the specific embodiments of the invention disclosed herein. Such equivalents are covered by the claims.

## EXAMPLES

### EXAMPLE 1: IN VITRO TESTING OF ENZYME PREPARATIONS

#### A. Purification of S. Marcescens Chitinase

A crude preparation of chitinase derived from cultures of S. marcescens (USBC lot #26102, with a specific activity of 148 nephalometric units per mg of protein) was purchased from United States Biochemical Corporation (Cleveland, OH). Protease assays using azocasein as a substrate (similar to assays described in Long 1981) indicated that this crude preparation was contaminated by substantial quantities of hydrolytic and other proteolytic enzymes which could be toxic to nematodes. Before an accurate assessment of chitinase effects on nematodes could be made, it was necessary to purify the crude preparation to remove any unspecific hydrolytic or proteolytic enzymes.

The first purification procedure involved affinity binding to purified colloidal chitin (similar to Roberts 1982). Briefly, this procedure involves the affinity binding of chitinase to colloidal chitin, which is insoluble. Initial binding occurs at 4°C, which inhibits cleavage of the chitin by the chitinase. Unbound enzymes and other soluble substances are removed from the solution by centrifuging the colloidal chitin and bound chitinase into a pellet, discarding the supernatant, and resuspending the pellet. The suspended pellet is incubated at 30°C to allow the chitinase to degrade and solubilize the chitin, which releases and solubilizes the chitinase for further purification. This procedure is described in detail below.

One gram of S. marcescens chitinase was suspended in 200 ml of 50 mM potassium phosphate buffer, pH 6.3 and dialyzed against 4 l of the same buffer, at 4°C. After an initial 12 hr dialysis, the buffer was replaced with 4 l of fresh buffer and dialyzed an additional 3 hr. The protein concentration, measured by the method of Bradford 1976 using a bovine serum albumin standard, was diluted to a predetermined concentration of 0.67 mg protein per ml, a concentration optimized for the binding and elution of chitinase to colloidal chitin. An equal volume of purified colloidal chitin slurry (7 mg of chitin, dry weight/ml in 50 mM $KPO_4$ pH6.3), synthesized as described by Molano 1977, was added to the dialyzed, diluted chitinase preparation at 4°C. The slurry was suspended by shaking by hand for 30 sec at 15 min intervals to assure uniform binding of the chitinase to the colloidal chitin. Samples removed periodically were centrifuged for two min in an Eppendorf microfuge and assayed for chitinase binding by measuring the solubilization of radioactive chitin (Molano 1977), which indicated the amount of chitinase activity which remained unbound in the supernatant. Binding was rapid and complete within 15 min.

Chitin-bound chitinase was centrifuged for 20 min at 20,000 g to sediment the complex. The pellet was washed with 900 ml of 50 mM potassium phosphate buffer, pH6.3, then suspended in the same buffer and incubated at 30°C to allow the chitinase to degrade the chitin, which releases and solubilizes the chitinase. Approximately 30% of the bound chitinase activity elutes from the chitin after 24 hr incubation at 30°C. The chitinase was centrifuged as above to remove particulates, and dialyzed for 4 hr, then 18 hr against 4 1 of 50 mM potassium phosphate buffer, pH 6.3 to remove chitin degradation products. Dialyzed chitin was concentrated 20 fold in an Amicon concentrator. The resulting mixture is believed to contain five distinct forms of chitinase.

Nonspecific protease activity was measured using azocasein (Sigma Chemical Co., St. Louis, Missouri) as the substrate, as described by Long 1981, except that protease activities were measured at pH 6.3 in 50 mM potassium phosphate buffer. Nonspecific protease activity was reduced approximately 150 fold relative to the purchased chitinase preparations by the chitin affinity purification step, and to an undetectable extent by the molecular sieve chromatography. Therefore, results obtained from nematocidal testing (Example 1.B) using the purified chitinase can be attributed to chitinase without reservation concerning nonspecific proteases or other hydrolytic enzymes.

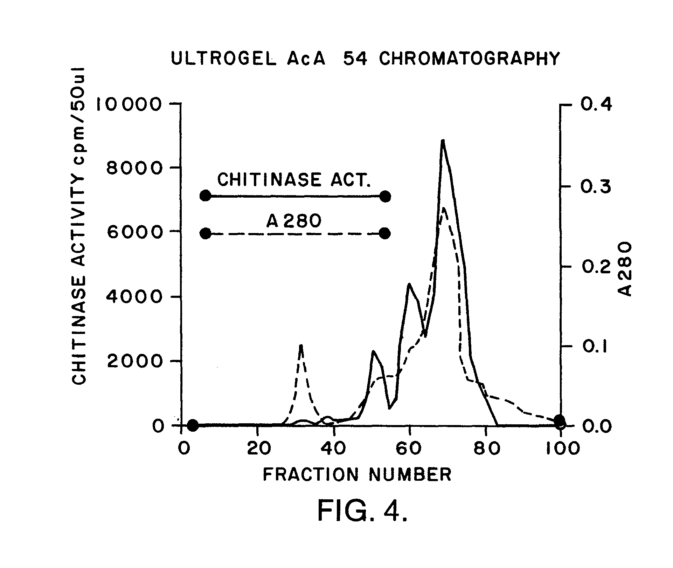

Approximately 4 to 9 mg of concentrated chitinase was loaded onto a 1.5 cm x 95 cm (168 ml bed volume) column (Ultrogel AcA54, LKB Instruments, Rockville, MD) and eluted at approximately 10 ml/hr. 1.5 ml fractions were collected and analyzed for chitinase activity, which separated into 3 distinct peaks (Fig. 4), each with unique monomeric molecular weights as determined by SDS-PAGE analysis. The majority of chitinase activity and total protein eluted in the final peak as two unique proteins of different monomeric molecular weight. Electrophoresis of these proteins under non-denaturing conditions revealed two protein bands with molecular weights of about 57 and 21 Kd, both possessing chitinase activity. These two chitinase species have been purified to >95% purity based on visualization of SDS-PAGE gels stained with Coomassie blue.

## B. Chitinase Treatment of M. Javanica Eggs

Eggs of the root-knot nematode, _Meloidogyne javanica_, were obtained by homogenization in water of infected tomato roots six weeks after inoculation of greenhouse potted plants with L2 stage larvae.

The root homogenate was sequentially sieved using a 60 mesh screen, a 200 mesh screen and a 500 mesh screen (W. S. Tyler Co., Mentor, OH). Mesh size indicates the number of holes per square inch. Eggs are retained by the final sieve (for example, 500-mesh eggs will pass through a 200 mesh screen but not through a 500 mesh screen) and transferred to a 50 ml conical tube for centrifugation. 40 ml of an aqueous egg suspension was pelleted after 4 min at setting number 6 of a Dynac table top centrifuge (Becton, Dickinson and Company, Parsippany, NJ). The supernatant fluid was discarded and the eggs resuspended in a 50% w/v aqueous sucrose solution. Centrifugion of this suspension under the previous settings permitted collection of eggs by flotation in the sucrose solution.

Eggs were recovered by gentle shaking on an ethanol-washed 500 mesh sieve which had been previously sterilized under short-wave length U.V. light overnight. Eggs were surface-sterilized by suspension in 50 ug/ml of streptomycin sulfate (Sigma Chemical Co.) and incubation at room temperature for 4 hrs. To remove the antibiotic after surface sterilization, the eggs were placed in the sterile sieve and washed three times with sterile water.

Eggs were distributed in sterile culture tubes. Potassium phosphate pH6.3 was added to a concentration of 50 mM. Each 1 ml aliquot contained approximately 9200 eggs.

Different batches of eggs were immersed in $KPO_4$ buffer (controls), or with buffer containing 1, 10, or 100 ug/ml of the mixture of five S. marcescens chitinases, purified as described in Example 1.A. As a control for the possible effect of protein concentration, bovine serum albumin at the same concentrations was also prepared and tested; no significant effect was observed. Each treatment was conducted on four batches of eggs, and triplicate samples (25 ul volume each) from each replicated treatment provided 12 samples. Emerged larvae (motile and non-motile) were counted under a stereomicroscope (40X) at 24 hr intervals over a 10 day period. Typical beginning egg counts per 25 ul sample were 231 ± 31 (n=36 treatment tubes).

0171381

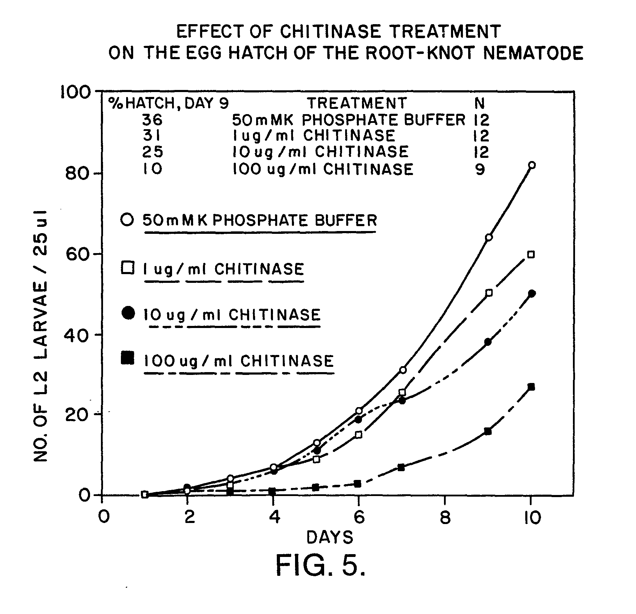

The results, presented in Figure 5 and in Table 4, indicate that chitinase at a concentration as low as 1 ug/ml inhibits the emergence of larvae from nematode eggs.

## TABLE 4
### EFFECTS OF CHITINASE ON NEMATODE EGGS

| Treatment | Egg Hatch After Ten Days (Mean ± St.dev.) |
|---|---|
| Buffer only (controls) | 82.2 ± 10.15 |
| 1 ug/ml chitinase | 53.1 ± 15.7 |
| 10 ug/ml chitinase | 49.9 ± 13.8 |
| 100 ug/ml chitinase | 26.0 ± 4.6 |

## C. Treatment of Larvae With B-gal, Mannosidase

Freshly hatched M.javanica larvae were immersed for 24 hr in 300 units/ml B-gal (from E. coli), 1 mg/ml mannosidase (from jack bean), or 1 mg/ml bovine serum albumin (BSA, as a control). The larvae were inoculated onto the roots of two week old tomato plants, and galls were counted 14 days later. The results, shown in Table 5, indicate that the B-gal treatment did not affect the larvae to a large degree compared to the control, but that the mannosidase treatment reduced galling by about 44%.

## TABLE 5

Effects of 24 hr Treatment of <u>M.javanica</u> Larvae by
B-gal or Mannosidase

| Treatment | Gall count at 14 days (mean ± st. dev.) |
|---|---|
| BSA (control) | 40.4 ± 9.5 |
| B-gal | 37 ± 11.9 |
| Mannosidase | 22.8 ± 6.3 |

## EXAMPLE 2: MANIPULATION OF CHITINASE GENE FROM S. MARCESCENS

### A. Isolation and Partial EcoRI Digestion of S. Marcescens Chromosomal DNA

A strain of <u>Serratia marcescens</u> designated as QMB1466, was obtained from E. Cabib (National Institutes of Health). This strain was originally characterized by Monreal 1969, and is reported to actively secrete chitinase. One liter of S. marcescens QMB1466 was grown in Luria Broth (LB) at 30° C to an A600 of 2.0, and the cells were washed with 0.15 M saline, 1 mM EDTA and frozen at -70°C. Chromosomal DNA was isolated as described by Marmur 1961 except that the DNA preparation was subjected to a phenol extraction after the RNase treatment and preceeding the chloroform extraction. Approximately 1 mg of chromosomal DNA was isolated. Twenty micrograns (ug) of this DNA was partially digested for 15 min at 37°C with 8 units of EcoRI (all endonucleases were obtained from New England Biolabs, Beverly, MA or from BRL, Gaithersburg, MD) in a 150 ul reaction mixture containing EcoRI buffer as described by the manufacturer.

These conditions were designed to optimize recovery of 18 to 28 kb fragments of chromosomal DNA. EcoRI digestion was terminated by heating at 65°C doe 5 min. Partially digested DNA was precipiated with 2 volumes of ethanol in the presence of 0.25 M sodium acetate, pH 5.2 and suspended in TE buffer (10 mM Tris-HCl, 1 mM EDTA, pH 8.0) at 0.5 ug/ul.

## B. Insertion of S. Marcescens DNA Into Cosmid Vectors

Cosmid pRMSL26 (Long 1981) was obtained from F. Ausubel, Harvard University. This cosmid, which contains about 20 kb of DNA from a Rhizobium species, was digested with EcoRI and religated to reconstitute a 21.6 kb cosmid believed to be comparable to pLAFRl (Friedman 1982).

The 21.6 kb cosmid was transformed into $CaCl_2$-shocked E. coli HB101 cells (ATCC #33,694). Cells were grown in 1 liter LB medium containing 12.5 ug/ml tetracycline (Tc) overnight to saturation. 160 ml aliquots of these cells were harvested by centrifugation, washed with cold TE buffer, and stored as frozen pellets. The cosmid DNA was prepared using "Procedure A," as follows.

Pellets were thawed and suspended in 6 ml TEG buffer (25 mM Tris-HCl, pH8, 50 mM EDTA, and 1% w/v glucose) and twelve ml of alkaline SDS (10.5 ml distilled water, 3.0 ml 1 N NaOH, 1.5 ml 10% sodium dodecyl sulfate) was added, mixed by inversion, and placed on ice for 20 minutes. Nine ml of ice cold KOAC (60 ml 5M potassium acetate, 11.5 ml of glacial acetic acid, 28.5 ml distilled water) was added, mixed by inversion, incubated for 10 min. on ice, and spun at 12,000 g for 10 minutes. Approximately 20 ml supernatant was transferred into two new tubes (10 ml each). An equal volume of phenol:chloroform (1:1 using TE buffer-saturated phenol) was added, vortexed, and spun at 12,000 g for 10 minutes.

Approximately 10 ml of the upper phase was transferred into each of two new tubes. Twenty ml ethanol (2 volumes) was added to each tube, precipitated for 20 min on ice, and centrifuged at 10,000 g for 10 min. The pellet was rinsed in ethanol and dried for 5 min under vacuum. One ml TE buffer was added to each tube, and the DNA was resuspended and combined into one tube (2 ml total).

RNase A (Sigma Chemical Co., St. Louis, MO) was prepared to 10 mg/ml in 10 mM Tris-HCl (pH 7.5) with 15 mM NaCl. The RNase A was boiled 10-15 minutes to inactivate contaminating DNase, and cooled slowly to room temperature. Aliquots were stored at -20°C.

One hundred ul RNase A (10 mg/ml) and 1-2 ul RNase T1 (approximately 300 u/ul; BRL) were added and incubated 15 minutes at 37°C. 500 ul 2M NaCl/TE buffer was added. The mixture was loaded directly onto an equilibrated NACS-52 mini-column, prepared as described below.

NACS-52 resin was hydrated following the manufacturer's instructions (BRL), and stirred on a magnetic stirrer. A 22 gauge needle was attached to a 10 ml Econo-column (Bio Rad, Richmond, CA) and P90 intramedic tubing was attached to the needle. 6 ml of resin (approx. 0.4 g) was pipetted into each column, and most of the resin buffer (2 M NaCl/TE) was allowed to flow through the column. Ten ml 0.4 M NaCl/TE was added and allowed to flow through before the sample was added.

The DNA sample (2.6 ml total) was loaded onto the column. Most of the DNA adhered to the NACS-52 resin during this step. To improve the yield, the eluant was collected and run through the column 3 times. The column was washed with 30 ml 0.4 M NaCl/TE to remove unbound protein and RNA.  The DNA was eluted with two 1 ml aliquots of 0.65 M NaCl/TE.

Two ml ethanol was added to each aliquot and precipitated on dry ice for 10 minutes, spun for 5 min in a microfuge, decanted, rinsed with ethanol, dried under vacuum for 5 min, and resuspended in 200 ul TE buffer.

The yield and authenticity of the DNA was determined by reading the optical density (OD260) and by analysis of EcoRI and BglII restriction fragments on a 0.8% agarose gel.

1.5 ug of purified DNA was restricted for 60 minutes at 37°C with EcoRI and treated with calf-intestine alkaline phosphate (CAP).  The resulting fragments were combined with 0.5 ug of purified S. marcescens DNA which had been partially digested with EcoRI as described in Example 1.  T4 DNA ligase in buffer containing 1 mM ATP was added and the mixture was incubated at 23°C for 40 minutes then at 16°C for 40 minutes.


## C.   Cloning of S. Marcescens DNA

Bacteriophage packaging extracts can be purchased from Promega Biotech (Madison, Wisconsin). However, for the experiment described herein, they were prepared basically as described by Maniatis 1982. Briefly, this procedure involved the use of two complementary E. coli cell lines, each of which contains a lambda prophage sequence with a single defective gene.  Cell line BHB2688 contains a prophage with a defective "E" gene, which cannot produce the E protein for the phage capsid.  Cell line BHB2690 contains a prophage with a defective "D" gene. Prophages from both cell lines were induced to express phage proteins, and the proteins were mixed to assemble complete phage capsids. The cosmids with S. marcescens inserts were mixed with the phage capsids. Because of phage packaging characteristics, only those cosmids having inserts of about 22 to 26 kb were inserted into these capsids to make phages which can infect E. coli cells. The inserted cosmid DNA replicates in E. coli cells, but since it does not encode a full set of phage proteins, it functions like a plasmid rather than a phage inside the E. coli cells.

To prepare the freeze/thaw lysate, E. coli BHB2688 cells were grown at 30°C overnight in LB medium to saturation. Cells were subcultured in 500 ml LB medium in a 2 liter flask and shaken at 30°C until the culture density reached 75 Klett units. Prophage replication was induced by heating over a flame to 45°C for 15 min, then incubation for 2.5 hours at 39°C.

Cells were centrifuged at 10,000 g for 10 minutes at 4°C. The pellet was resuspended in 1 ml of cold distilled water containing 20% w/v sucrose, then aliquoted into 500 ul fractions. 25 ul of 2 mg/ml lysozyme in 250 mM Tris-HCl (pH 8) was added to each fraction and mixed gently. The tubes were then capped and submerged in liquid nitrogen for 2-3 minutes, then placed on ice and allowed to thaw for 1 hour. 25 ul of packaging buffer (6 mM Tris-HCl (pH8), 50 mM spermidine, 50 mM putrescine, 20 mM $MgCl_2$, 30 mM ATP, 30 mM 2-mercaptoethanol) was added to each tube, and the extracts were combined. The mixture was centrifuged at 30,000 g for 25 min at 4°C. The supernatant was dispensed in 50 ul aliquots and stored at -80°C.

Sonication extracts were prepared by culturing E. coli BHB2690 cells by the same procedure as for BHB2688 cells. After centrifugation, the pellet was resuspended in 3.6 ml cold sonication buffer (20 mM Tris-HCl (pH 8); 1 mM EDTA; 5 mM 2-mercaptoethanol) and lysed by sonication. Lysates were microfuged for 10 min at 4°C, and the pellets were discarded. An equal volume of sonication buffer and 1/6th volume of fresh packaging buffer were added to the supernatant, and mixed. The mixture was dispensed into 50 ul aliquots and stored at -80°C.

5 ul of the ligated cosmid S. marcescens DNA was mixed with 10 ul of the sonication extract and 25 ul of the freeze-thaw lysate, incubated at 23°C for 60 minutes, then mixed with an additional 210 ul phage buffer (50 mM Tris-HCl pH7.5, 0.1 M NaCl, 8 mM $MgSO_4$). 150 ul of the diluted, packaged cosmid DNA was used to infect E. coli SR193 cells (SR1257 C600 strA $r^-m^+$ leu$^-$pro$^-$suII [Lambda cI857 nin$^+\Delta$ BamHI 58-71 plac]) which had been grown at 30°C in LB medium containing 0.4% maltose to a density of 200 Klett units. After incubation of the cells and phage at 30°C for 20 minutes, 1 ml of LB medium was added, and the mix was incubated with shaking 70 min at 30°C. Cells were then concentrated by brief centrifugation, plated on LB plates containing 14 ug/ml Tc, and incubated at 30°C for 48 hours.

## D. Screening for Chitinase in E. Coli SR193

Transformants from Example 2.C were screened for chitinase activity using agar plates containing translucent colloidal chitin. Tests involving S. marcesens cells indicated low sensitivity of commercially available chitin, so an improved method for creating chitin plates was developed, as follows.

Chitin was synthesized using chitosan (Sigma Chemical Co.) using the method of Molano 1977. Extensive washing of the chitinase was essential; it was washed several times in distilled deionized water, then the pH was adjusted to 7.0, and the mxiture was washed several more times. The synthesized chitin was homogenized using a Polytron blender (Brinkman Instruments; Westbury, New York) before autoclaving. These steps rendered the chitin at least 10X more sensitive to chitinase degradation than the commercial preparations.

Plates containing an overlay of chitin (rather than chitin dispersed throughout the agar) were used. A 25 ml agar layer (0.1% w/v each of nutrient agar and agar (Difco, Detroit MI) and 12.5 ug/ml of Tc) was poured into Petri dishes. When the agar solidified, 10 ml of the same agar media containing 5 mg/ml (dry weight) of colloidal chitin was added to each Petri dish. This increased the sensitivity of the clearing zone assays by about 3 to 5 fold.

Approximately 2800 $Tc^R$ clonal colonies (pLAFR1 contains a $Tc^R$ gene) were transferred onto the chitin overlay plates, using toothpicks. The plates were incubated at 30°C for at least 3 days before visual analysis. Six chitinase positive clones were identified by the presence of clear zones around the colonies, visible against the translucent background of the colloidal chitin. Two clones contained cosmids, designated as pMON5031 and pMON5032, which appeared to contain identical 24 kb inserts, as determined by EcoRI and BglII digestion. A third clone (pMON5033) contains the same insert minus a 1.7 kb EcoRI fragment. Four clones (pMON5027, 5030, 5031, and 5034) express chitinase activity in E.coli SR193.

E. coli clones were cultured overnight at 30°C in LB media containing 14 ug/ml Tc, and plasmid DNA was prepared by the following method ("Procedure B").

Cells were centrifuged for 2 minutes in an Eppendorf microfuge, decanted, resuspended in 100 ul TEG buffer, and vortexed until the suspension was uniform. 200 ul alkaline SDS (0.2 N NaOH, 1% SDS) was added, mixed by inversion, and put on ice for 5 min. 150 ul of potassium acetate (KOAC) mixture (3 M KOAC + 2 M acetic acid) was added, mixed by inversion, put on ice for 5 min, and spun in a microfuge for 5 min. 450 ul (or less) was decanted into an Eppendorf tube. 450 ul phenol/chloroform (1:1 TE buffer-saturated phenol + chloroform) was added, vortexed, and microfuged for 2 min. The top layer (approx. 350 ul) was transferred to another Eppendorf tube. An equal or greater volume of anhydrous ether was added and vortexed until the lower phase was relatively clear. The upper phase was decanted and discarded. The ether treatment was then repeated.

700 ul ethanol was added, incubated on ice for 15 min, microfuged for 5 min, and decanted. 250 ul TE buffer was added, and the mixture was vortexed to dissolve the pellet. 25 ul 3M sodium acetate (pH 4.8) was added and mixed. 600 ul ethanol was added and mixed. The mixture was incubated 10 min on dry ice, microfuged for 5 min, and decanted. 500 ul ethanol was added, and the mixture was microfuged for 1 min. The supernatant was decanted and discarded. The lower phase was dried under vacuum for 5 minutes, and resuspended in 17 ul distilled water.

2 ul 10X restriction buffer and 10-15 units of restriction endonuclease were added and incubated for 30 min at 37°C. 1 ul of RNase A (10 mg/ml was added to each sample. The mixture was incubated 5 min at room temperature and 2 ul 10X bromophenol buffer (50% glycerol and 0.002% w/v dye) was added. The sample was loaded onto an agarose gel which included a size marker. The gel was run until the bromophenol dye front reached the bottom of the gel. Agarose gels were stained with 1 ug/ml ethidium bromide (EtBr) for 20 min, destained in distilled water for 30 min, and photographed.

Plasmid DNA was restricted with EcoRI, BglII, HpaI, BamHI, SmaI, or SphI, and the gel band patterns compared. These indicated that all positive clones contained a common 9.5 kb sequence having EcoRI sites at each end but no internal EcoRI sites.

E. coli LE392 cells containing pMON5030 or 5027 were cultured for 10 days at 30°C in LB medium. The cells were lysed by sonication, centrifuged to remove particulate matter and dialyzed overnight in 50 mM $KPO_4$ buffer. This resulted in a crude protein preparation which was purified by the methods described in Example 1.A. Analysis on an SDS-PAGE gel indicated that the chitinase expressed by the transformed E. coli was a monomeric protein with a weight of about 57 kilodaltons. A 57 kd protein would require a coding sequence about 1.6 kb long.

Creation of cleared zones of degraded colloidal chitin is dependent on the release of chitinase by the transformed E. coli cells into the media. E. coli SR193 contains a defective temperature sensitive lambda prophage; this strain was chosen so that cell rupture by phage lysis could be induced if necessary by heating the cultures to 42°C. Induced cell rupture was found to be unnecessary, since sufficient chitinase was being released by uninduced cultures to create chitin clearance zones around certain colonies.

To analyze chitinase release by E. coli cells, three of the vectors (pMON5027, 5032 and 5034) were isolated by "Procedure A" described in Example 2.B, then transformed into a different E. coli strain, LE392 (Maniatis 1982), using standard $CaCl_2$ procedures. E. coli strain LE392 does not contain any lambda lysogen.

All three transformed colonies of LE392 created chitin clearance zones that were comparable to the clearance zones measured in SR193 cells.  This indicated that E. coli secreted chitinase from S. marcescens utilizing the natural secretion process which allows secretion of chitinase from viable, untransformed S. marcescens cells.  However, subsequent experiments indicated that little if any chitinase was actively secreted by viable cells, as described in Example 2.H.

E. Transfer of Chitinase-Coding Cosmids To Pseudomonas 701E1

Cosmids from the six positive chitinase-producing clones were used to transform the soybean root-colonizing Pseudomonas fluorescens 701E1 (ATCC #39773) by a tri-parental mating using E. coli cells containing plasmid pRK2013 (see Figurski 1979).  Colonies containing the cosmids were verified by restriction pattern analysis (using "Procedure B" described in Example 2.D), and streaked onto chitin overlay plates as described in Example 2.D.  The plates were incubated at 30°C for 3-10 days, and observed for clearing of the chitin background.  It was noted that some of the clones which cleared the chitin when contained in E. coli SR193 also cleared chitin in Ps. 701E1, including pMON5031, pMON5032, pMON5034, and pMON5027.  However, the size of the zones surrounding the Ps. 701E1 colonies, and the rate at which they appeared were generally lower than the corresponding characteristics in E. coli.  A culture of Ps. fluorescens 701E1 containing cosmid pMON5031 has been deposited with the ATCC, and has been assigned accession number 39765.

## F. Subcloning Of Chitinase Gene and Expression
## From Strong, Constitutive Promoters

E. coli SR193 cells containing cosmid pMON5031 were cultured to saturation in 400 ml LB medium containing 14 ug/ml tetracycline at 30°C. Cells were collected by centrifugation and plasmid DNA was purified by Procedure A (Example 2.B). The authenticity of this DNA was verified by restriction pattern analysis using EcoRI, HpaI, SmaI, SphI, and/or BglII as described above. Complete digestion of pMON5031 with EcoRI created three small fragments, and the large cosmid fragment.

Similarly, plasmid pMON5012 DNA was transformed into and purified from E. coli M182 (lac⁻). 1.2 ug of pMON5012 DNA and 2.4 ug of pMON5031 DNA were digested (separately) to completion with EcoRI and concentrated by ethanol precipitation. The DNA fragments were mixed, incubated with T4 DNA ligase and used to transform E. coli MC1009 (lac⁻, recA⁻) that had been treated with CaCl₂. Transformants were selected on LB plates containing 50 ug/ml kanamycin and 64 ug/ml X-gal, after growth overnight at 37°C. White colonies were transferred onto chitin overlay plates and allowed to grow at 37°C for 3 days. Clearing zones were found around more than 25% of the colonies, indicating that one of the three EcoRI fragments contained at least one chitinase gene, and that strong expression of this gene could be obtained in either orientation (from either the Pbla or the Pori promoter).

Plasmid DNA was purified from eleven positive candidates using Procedure B (Example 2.D), and the EcoRI restriction patterns analyzed. All carried a 9.5 kb EcoRI fragment. In addition, restriction with BglII was carried out on two of the eleven isolates which appeared to have slightly larger zones of clearing on the chitin plates, and on two more which had smaller zones. The resulting patterns indicated that the two candidates with larger zones of chitin clearance were oriented in one direction with respect to a unique BglII site on the plasmid (which was designated as pMON5036). The two other candidates with smaller zones of chitin clearance were oriented in the reverse direction. Since the Pbla promoter is generally stronger than Pori, it was inferred that the orientation of the chitinase gene in pMON5036 is under the control of the Pbla promoter, as shown in Fig. 2. One of the clones with the chitinase gene orientated in the reverse direction was designated pMON5035. A culture of E. coli MC1009 cells carrying pMON5036 has been deposited with the ATCC, and has been assigned accession number 39764.

## G. Creation of pMON5040

Plasmid pMON5036, prepared as described in Example 1.F, was removed from E. coli MC1009 cells using Procedure A, described in Example 2.B. 5 ug of DNA was suspended in 30 ul of EcoRI buffer containing 20 units of EcoRI, and digested for 30 min at 37°C. The EcoRI was inactivated by heating to 70°C for 15 min. The DNA was precipitated by ethanol, and resuspended in 30 ul of BglII buffer with 24 units of BglII. The mixture was digested for 30 min, precipitated with ethanol, and suspended in 15 ul of distilled deionized water. 2 ul of 10X ligation buffer, 2 ul of 10 mM ATP, and 1 ul of T4 DNA ligase were mixed, added to the DNA suspension, and incubated overnight at 16°C. The ligated DNA was used to transform $CaCl_2$-shocked E. coli MC1009 cells. The cells were incubated 1 hr in LB at 37°C, plated on LB containing 50 ug/ml kanamycin, and incubated overnight at 37°C.

200 colonies were selected and plated onto chitin overlay plates. After 2 days, 32 colonies were surrounded by clearing zones, and 12 colonies were selected. 5 of these colonies were surrounded by relatively large clearing zones, and the remaining 7 were surrounded by smaller clearing zones. Plasmids were removed from each colony by Procedure B (Example 2.D). Half of the DNA was digested with BglII only, and the other half of the DNA was digested with EcoRI and BglII simultaneously. The DNA fragments were analyzed on a 0.8% agarose gel. This analysis indicated that the 7 clones with weak chitinase expression had a 3.8 kb insert. One of these plasmids was designated as pMON5039.

The 5 clones with strong chitinase expression had a 2.4 kb insert. The plasmid in one of these clones was designated pMON5040, and was analyzed using endonucleases EcoRI, BglII, PstI, and SmaI. The digestion patterns were compared with digestion patterns from the 3.8 kb insert in pMON5039. The results indicated that the 2.4 kb insert was contained entirely within the 3.8 kb insert. This was unexpected, since no EcoRI or BglII cleavage sites existed within the 3.8 kb insert. This indicated the occurrence of "star" cleavage, i.e., cleavage by an endonuclease of a DNA sequence other than its normal cleavage sequence (see, e.g., Fuchs 1983).

Star cleavage can be induced by manipulation of endonuclease digestion conditions (primarily temperature, salt and glycerol concentration, and pH), but star cleavage is often unpredictable and unreproducible, and no effort was made to induce star cleavage of pMON5036. Its occurrence during the digestion of pMON5036 was unexpected and fortuitous, and the creation of perfect (GAATTC) EcoRI cleavage sites at both ends of the 2.4 kb insert of pMON5040 was also unexpected and fortuitous.

pMON5040 was inserted into a strain of Ps. fluorescens 701El via triparental mating, and the cells were plated on chitin overlay plates. A substantial chitin clearing zone was observed in less than 48 hours, which was faster than the clearing of chitin caused by other transformed strains of pseudomonads. A culture of E. coli LE392 cells containing pMON5040 was deposited with the ATCC, and was assigned accession number 39774.

The 2.4 kb insert was removed from pMON5040 by EcoRI digestion and inserted into plasmid pUC8 (Vieira 1982), under the control of the lac promoter, which is inducible by IPTG. The resulting plasmid, designated as pMON5042, was transformed into E. coli JM101 cells, which were plated onto chitin overlay plates containing an overspread of IPTG. These cells created chitin clearing zones within less than 8 hours.

## H. Testing of Chitinase Secretion

E. coli cells containing pMON5040 were grown overnight at 30°C in Luria broth containing 50 ug/ml kanamycin. Cells from that culture were used to inoculate 100 ml of fresh media, to a density of 5 to 10 klett units. These cultures were grown to a density of 100 klett units (about 5 hours). Samples were taken and centrifuged for 20 minutes at 20,000 g. Supernatants were decanted, concentrated 10X in a Minicon concentrator (Amicon, Danvers, Massachusetts), and analyzed as described below to estimate secreted chitinase.

Each cell pellet was suspended in 2.0 ml of 50 mM potassium phosphate, and lysed by passage twice through a French pressure cell (Aminco, Silver Springs, Maryland) at 20,000 psi. Crude cellular extracts were centrifuged at 20,000 g for 30 minutes at 4°C to remove particulates, and the resulting supernatant was used to estimate intracellular chitinase.

Intracellular and secreted chitinase concentrations were determined using $^3$H-labelled chitin, as described by Molano 1977, using 10:1 dilutions if the results exceeded the linear range. Intracellular chitinase (extracted from the centrifuged pellet) created 0.85 micromoles of NAG per minute, while secreted chitinase (from the initial supernatant) created 0.08 uM of NAG under comparable conditions. In a second run, the results were 1.2 uM (intracellular) and 0.08 uM (secreted) of NAG. These results indicated that only about 6 to 8% (or less) of the total chitinase activity was present in the supernatant after centrifugation of the cells at 20,000 g for 20 minutes. This figure is substantially less than is present for most actively secreted proteins, and is likely to be caused by cell rupture during the culturing and centrifugation steps. Nevertheless, as shown in Example 3, release of S. marcescens chitinase by transformed pseudomonads over sustained periods (e.g., 28 days), presumably by ruptured cells, was sufficient to inhibit nematodes.

EXAMPLE 3: NEMATODE INHIBITION BY TRANSFORMED CELLS

A. Preparation of Bacterial Inoculum

Several strains of Ps. fluorescens were used. Strain 1141 is a spontaneous rifampicin $^R$ mutant (isolated on 50 ug/ml rif) of a strain was

isolated from soybean roots near Sun Prairie, Wisconsin, Strain 112-12 is a rif$^R$ mutant of a strain isolated from corn roots in St. Charles County, Missouri. Strain 701E1 is a rif $^R$ mutant of a strain which was isolated from soybean roots near Hoopeston, Illinois. Rifampicin resistance occurs naturally in Ps. fluorescens, and can be selected for without inserting any foreign genes by contacting the cells with rifampicin. It is not believed to have any effect on nematode inhibition.

Modified versions of all three of these strains were created by inserting chimeric lacZY genes into their chromosomes. This was done by a bicomponent transposition system similar to the system described in EPO 91,723 (Grinter; Imperial Chemical Industries). Briefly, that system involves :

1. a helper plasmid which contained the genes for the Tn7 transposase enzymes, tnpA, B, and C, and a Kanamycin $^R$ gene; and

2. a carrier plasmid with a gentamycin $^R$ gene, and with the lacZY gene located between the termini of the Tn7 transposon, but without any transposase genes.

The transposase enzymes expressed by the helper plasmid allowed the modified transposon to be transferred from the carrier plasmid into the chromosomes. The helper plasmid was then removed, and the absence of transposase genes in the progeny cells ensures that the inserted lacZY gene cannot be transposed out of the chromosomes.

The chimeric lacZY gene contained coding sequences for lacZ (which encodes B-gal) and lacY (which encodes lactose permease), both regulated by a strong constitutive promoter, the Pbla promoter, derived from a beta-lactamase gene.

When transformed with the chimeric lacZY gene, the strains 112-12, 1141, and 701E1 used in subsequent tests were designated as 112L1, 1141L1 and 701L5, respectively.

All bacterial cultures were grown to 300 Klett for 24 hours in M9 medium with glucose (3g $KH_2PO_4$, 7g $Na_2HPO_4$, 1g $NH_4Cl$, 0.5g NaCl, 10g glucose, 25 ul 1M $CaCl_2$, 1.2 ml 1M $MgSO_4$ $7H_2O$ per liter distilled deionized $H_2O$). The glucose, $CaCl_2$, and $MgSO_4$ were added after autoclaving. Antibiotics for selections were added in the following concentrations: rifampicin (50 ug/ml), kanamycin (50 ug/ml).

30 ml of the culture used for any treatment (indicated in each table) were centrifuged for 10 minutes at 5000 g and resuspended in M9 glucose without antibiotics. One ml of resuspended bacteria was applied by pipette to each seed. Table 6 indicates the number of bacteria inoculated onto the seeds at the start of each test; these data are based on three dilutions ($10^{-5}$, $10^{-6}$, $10^{-7}$) with two reps each on modified M9 agar (MM9), which substitutes 0.5 g sodium citrate for 0.5 g NaCl.

TABLE 6

Number of Bacteria Contained in Inocula

| Isolate | Foreign Genes | Mean CFU/ml M9 glucose |
|---|---|---|
| 1141L1 | lacZY | $6.2 \times 10^9$ |
| 1141L1/pMON5040 | lacZY, chitinase, kanR | $6.9 \times 10^9$ |
| 112L1 | lacZY | $2.0 \times 10^8$ |
| 112-12 | | $1.3 \times 10^9$ |
| 701L5/pMON5040 | lacZY, chitinase, kanR | $1.2 \times 10^{10}$ |

## B. Preparation of Containers

M. incognita eggs were collected using 400 and 500 mesh sieves. Approximately 1200 eggs, or mixed L1 and L2 larva, were mixed with 100 cc of the soil mix (equal parts WB10 sand, WB20 sand and unsterilized soil from St. Charles County, MO). The nematode inoculum (1200 eggs/10 ml $H_2O$) was added to the soil in a glass beaker, lightly mixed, and pooled with 10 samples in an automated mixer for 2 minutes. 100 cc of the soil nematode mix was then added to a conical plastic root container, which contained 10 ml of coarse vermiculite.

One soybean seed (Williams 82 cultivar) was added to each root container. One ml of the bacterial inoculum was applied to each seed and then covered with 3 cm of fine vermiculite. Plants were watered twice a day except as noted. After the primary leaves opened, a fertilizer solution (Peters Professional Water Soluble Fertilizer N=20, P=19, K=18; W. R. Grace Co., Fogelsville, Penn.; 1 tablespoon/gallon) was applied (during watering) once a day. The root container was maintained at 28°C.

## C. Harvesting of Roots for Population Counts

All plants were pulled from the pots and gently shaken to remove the majority of the soil mix from the root. Each root was severed at the soil surface and placed in a 50 ml centrifuge tube containing 30 ml of sterile water. These were shaken for 15 minutes at 200 rpm, and the roots were removed and placed into a second tube for a repeat washing. The water from the first washing was weighed for soil content. Two aliquots (50 ul each) of the first wash were diluted to $10^{-5}$ and $10^{-7}$ to determine the "rhizo- sphere population" of the soil, shown in Table 7. All dilutions were plated onto MM9 with 1% lactose (without glucose), 50 ug/ml cycloheximide, 40 ug/ml X-gal, and 50 ug/ml rifampicin; media for strains containing pMON5040 also contained 50 ug/ml kanamycin. The only exception was strain 112-12 which was selected on MM9 with glucose instead of lactose.

TABLE 7

Rhizosphere Populations for Bacterial Treatments

(CFU per gram soil)

| Isolate | # Days Post Planting | | | |
| | 4 | 11 | 22 | 28 |
|---|---|---|---|---|
| 1141L1 | $4.4 \times 10^6$ | $4.9 \times 10^6$ | $3.1 \times 10^6$ | $1.5 \times 10^6$ |
| 1141L1/pMON5040 | $1.3 \times 10^7$ | $8.9 \times 10^6$ | $4.6 \times 10^6$ | $2.0 \times 10^6$ |
| 112L1 | $5.9 \times 10^6$ | | $2.6 \times 10^6$ | $1.0 \times 10^6$ |
| 112-12 | $1.3 \times 10^7$ | | $1.3 \times 10^6$ | $7.6 \times 10^5$ |
| 701L5/pMON5040 | $4.0 \times 10^6$ | | | $4.9 \times 10^5$ |

The root was placed in 30 ml sterile water, and shaken for 15 minutes at 200 rpm. The root was then placed in a third tube for a final washing. The third washing, with 30 ml sterile $H_2O$, was also shaken for 15 minutes, and two aliquots (50 ul each) were diluted to $10^{-5}$ and $10^{-7}$ to determine the "rhizoplane population" counts, shown in Table 8.

## TABLE 8

Rhizoplane Populations for Bacterial Treatments
(CFU per gram fresh root)

| Isolate | # Days Post Planting | | | |
| | 4 | 11 | 22 | 28 |
| --- | --- | --- | --- | --- |
| 1141L1 | $7.3 \times 10^6$ | $1.6 \times 10^6$ | $2.9 \times 10^5$ | $5.6 \times 10^5$ |
| 1141L1/pMON5040 | $2.0 \times 10^7$ | $5.6 \times 10^6$ | $3.1 \times 10^5$ | $3.5 \times 10^5$ |
| 112L1 | $6.2 \times 10^6$ | | $2.8 \times 10^5$ | $1.2 \times 10^5$ |
| 112-12 | $4.8 \times 10^7$ | | $1.1 \times 10^5$ | $2.1 \times 10^5$ |
| 701L5/pMON5040 | $5.8 \times 10^6$ | | | $6.2 \times 10^4$ |

Root galls caused by nematodes were counted by hand under a lighted magnifying glass. The galls were easily distinguished from the nodules produced by rhizobium on soybean.

### D. Test No. 1

Sieved 500 mesh eggs were mixed with soil and placed in a root container. A soybean was planted in the container, and inoculated with one of the bacterial strains shown in Table 9. The container was watered twice and fertilized once each day. After 28 days, the roots were pulled up and treated as described above to determine nematode gall counts and bacterial populations.

The results of the tests involving the 1141 and 701 strains, shown in Table 9, indicate that after 28 days:

1. the 1141L1 strain with the lacZY gene inhibited nematodes, and when plasmid pMON5040 with a chitinase gene was added to 1141L1, the transformed strain inhibited nematodes even more; and,

2. the highest degree of control (more than 50%) was achieved by the 701 strain, which contained the B-gal and chitinase genes, despite the lower bacterial counts (Tables 7 and 8) for that strain after 28 days.

## TABLE 9

Soybean Nematode Gall Counts with 500-Mesh Eggs

| Bacterial Strain | Gall Count (Mean ± s.e.m.) | |
| --- | --- | --- |
| | 22 days | 28 days |
| Control (no inoculated bacteria) | 69.0 ± 5.3 | 104.9 ± 10.7 |
| 1141L1 (lacZY) | 46.0 ± 6.6 | 89.9 ± 6.9 |
| 1141L1/pMON5040 (lacZY, chitinase) | 33.0 ± 5.2 | 74.7 ± 9.3 |
| 701L5/pMON5040 (lacZY, chitinase) | - | 47.9 ± 9.7 |

The results of the test involving the 112-12 strain (which is normally a corn colonizer) with and without the lacZY gene are shown in Table 10. These results are not fully understood, since the 112-12 strain without the lacZY gene inhibited nematodes more effectively after 28 days than the 112L1 strain with the lacZY gene. It may be that those results are anomalous and would not recur if the experiment was repeated. Nevertheless, it is clear that the 112-12 strain, with or without the lacZY gene, substantially inhibited nematodes compared to the uninoculated controls.

TABLE 10

Soybean Nematode Gall Counts with 500-Mesh Eggs

| Bacterial Strain | Gall Count (Mean ± s.e.m.) | |
| --- | --- | --- |
| | . 22 days | 28 days |
| Control (no inoculated bacteria) | 69.0 ± 5.3 | 104.9 ± 10.7 |
| 112-12 | 46.6 ± 3.8 | 58.6 ± 6.3 |
| 112L1 (lacZY) | 44.6 ± 5.7 | 75.0 ± 9.7 |

E. Test No. 2

M. incognita eggs can be sieved to enrich for later or earlier developmental stages. "400-mesh" eggs do not pass through a screen having 62 holes per square centimeter (400 holes per square inch), with openings having a diameter of 0.038 mm (0.0015 inch). Those eggs are older and larger than 500-mesh eggs, and comprise approximately 65% eggs with vermiform shapes (L1 or L2). The 500-mesh eggs pass through a 400-mesh screen, but not through a screen having 500 holes per square inch with an opening diameter of 0.25 mm (0.0010 inch). These eggs represent the younger developmental stages; only 13% have vermiform shapes.

Root containers were inoculated with approximately 1200 400-mesh or 500-mesh eggs. A soybean was planted in each container, and bacteria were inoculated as described in Test No. 1. After 22 days the roots were pulled, washed free of soil, and the nematode galls were counted.

The results, shown in Table 11, indicate that chitinase does not inhibit nematode emergence from 400-mesh eggs; it is believed that chitinase may even promote the hatching of 400 mesh eggs by weakening the eggshell that encases larva which are ready to hatch. This factor is believed to account for some of the variability in the data obtained in Test No. 1. However, as indicated in Tables 9 and 12, chitinase substantially inhibits nematode emergence from 500-mesh eggs. Since bacterial inhibition of nematodes in the field will be exerted over extended periods involving numerous generations of nematodes, the ability of chitinase to damage young eggs should allow for effective nematode inhibition, regardless of its effects on ready-to-hatch eggs.

## TABLE 11

### Effects of Chitinase on 400 Mesh Eggs

| Inoculated Bacteria | Foreign Genes | Gall Counts (Mean ± St. error) 22 days |
|---|---|---|
| None (control | - | 88.8 ± 6.6 |
| 1141L1 | LacZY | 89.0 ± 3.1 |
| 1141L1/pMON5040 | LacZY, chitinase | 94.8 ± 8.7 |

The procedure described in Example 1.B was used to treat 400-mesh eggs of a different species, M. javanica, using S. marcescens chitinase from the same USBC lot (#26102) that was used in Example 1. The lyophilized unpurified chitinase (in vials supplied by USBC) had been stored for ten months at 4°C when this experiment was done. It was purified as described in Example 1.A to obtain the mixture of five chitinases. A portion of the purified chitinase mixture described in Example 1.A was also used, after storage for ten months at -80°C. Both mixtures were used to treat the 400-mesh nematode eggs as described in Example 1.B, except for the use of 25 mM $KPO_4$ buffer rather than 50 mM $KPO_4$. Although both of the stored chitinase mixtures showed the same activity on chitin in vitro, they did not significantly inhibit nematodes. In addition, a batch of 57 kd chitinase was purified from Ps. fluorescens 701E1 cells containing pMON5040; it did not significantly inhibit the 400-mesh eggs. These results confirm the result shown in Table 11.

## F. Test No. 3

Root containers were prepared with soil, 500-mesh nematode eggs, soybeans, and bacteria as described in Test No. 1, and grown for 28 days. Inadvertently, only one watering per day (containing fertilizer) was performed during part of the growing period.

The results, shown in Table 12, indicate that over a 28 day period, (which is effectively a single generation of nematodes):

1.  selected strains of pseudomonads with no foreign genes can effectively inhibit nematodes;

2.  soil bacteria which express B-gal can effectively inhibit nematodes; and,

3.  soil bacteria which express chitinase can effectively inhibit nematodes.

TABLE 12
NEMATODE INHIBITION BY PSEUDOMONADS
WITH B-GAL AND CHITINASE GENES

| Bacterial Strain | Foreign Gene | Gall Count (Mean ± s.e.m) |
|---|---|---|
| None (control) | - | 53 ± 2.8 |
| 1141L1 | LacZY | 31.8 ± 6.9 |
| 1141L1 (pMON5040) | LacZY, chitinase | 20.8 ± 2.6 |
| 701E1 | - | 32.6 ± 3.5 |
| 701L5 | LacZY | 14.8 ± 3.7 |
| 701L5(pMON5040) | LacZY, chitinase | 11.8 ± 1.9 |

REFERENCES

Bagdasarian, M., et. al., 1981, Gene 16:237-247

Bradford, M. M., 1976 Anal. Biochem. 72:248-254,

Carrick, L., 1973, "Purification and Partial Characterization of Ps. aeruginosa collagenase", Diss. Abstr. Int. B1974 34(11) (Univ. Microfilms #74-11, 088, Ann Arbor, MI).

Dropkin, V. H., 1980, Introduction to Plant Nematology (Wiley and Sons, New York)

Figurski, D. and Helinski, D., 1979, <u>Proc. Natl. Acad. Sci. USA 76</u>:1648

Friedman, A. M. et al., 1982, <u>Gene 18</u>:289-296

Fuchs, R. and Blakesley, R., 1983, <u>Methods Enzymol.</u> 100:3

Horwitz, M. et al, 1984, p.191-208 in Zikakis 1984

Ikeda, M. and Shimahara, K., 1979, "Purification of chitin...by a proteolytic bacterium, Ps. malto philia", <u>Nippon Nogei Kagaku Kaishi 52, 9</u>:355-360 (Japan)

Johnson, R.R., 1980, <u>Elementary Statistics,</u> Duxbury Press,     North Scituate, Massachusetts

Lee, D. L. and H. J. Atkinson, 1977, <u>Physiology of Nematodes</u> (Columbia University Press, New York

Long, S., et al, 1981, <u>J. Gen. Microbiol. 127</u>:193-199

Maniatis, T., et al, 1982, <u>Molecular Cloning (A Laboratory Manual)</u> Cold Spring Harbor Lab, New York

Mankau, R. and S. Das, 1969, <u>J. Nematology 1</u>:15-16

Marmur, J., 1961, <u>J. Mol. Biol. 3</u>:208-218

McClure, M. A., 1981, <u>Plant Parasitic Nematodes</u> 3:89-124.

McClure, M. A. and Zuckerman, B. M., 1982, <u>J. Nematology 14:</u> 39-44

Miller, J. H. and Reznikoff, W. S. 1980, <u>The Operon,</u> Cold Spring Harbor Lab, New York.

Miller, P. M. and D. C. Sands, 1977, <u>J. Nematology 9</u>:192-197

Molano, J.et al. 1977, <u>Anal. Biochem. 83</u>:648-656

Monreal, J. and E. T. Reese, 1969, <u>Canad. J. Microbiol. 15</u>:689-696

Nagahata, N. and Shimahara, K., 1979, "Properties of chitinase from Ps. aeruginosa E-139...", <u>Seikei Daigaku Kogakubu Kogaku Hokoku 27</u>:1909-10 (Japan)

Rao, R. S. S., et al, 1977, "Isolation of two facul tatively anaerobic organisms producing collagenase", <u>Indian J. Exp. Biol. 15, 9</u>:728-731

Reid, J. D. and D. M. Ogindziak, 1981, <u>Appl. Envirn. Microbiol. 41</u>:664-669

Roberts, R. L. and E. Cabib, 1982, <u>Anal. Biochem.</u>
<u>127</u>: 402-412

Schroth, M. N. and Hancock, J. G., 1981, <u>Ann. Review</u>
<u>Microbiol. 35:453-476</u>

Spiegel, Y. et al (1982A), <u>J. Nematology 14</u>: 33-35

Spiegel, Y. et al (1982B), <u>J. Nematology 14</u>: 406-407

Viera, J. and Messing, J., 1982, <u>Gene 19</u>: 259

Warnes, C. E. et al, 1984, <u>Abstracts Ann. Mtg. Amer.</u>
<u>Soc. Microbiol.</u>, #N20

Zikakis, J.T. (ed.), 1984, <u>Chitin, Chitosan, and Related</u>
<u>Enzymes,</u> Academic Press, Orlando Florida

0171381

CLAIMS

1. A method of inhibiting nematodes comprising inoculating the roots of a plant with cells from a selected strain of soil bacteria which is capable of:

a. proliferating on the roots of the plant in one or more types of soil under conditions of microbial competition; and

b. inhibiting one or more types of nematode when present on the roots of the plant.

2. A method of Claim 1 wherein the strain of soil bacteria is selected from the group consisting of Ps. fluorescens 701L5 (ATCC No. 53175) and Ps. fluorescens 1131L1 (ATCC No. 53176).

3. A method of Claim 1 wherein the bacteria express a foreign gene which encodes chitinase.

4. A method of Claim 3 wherein the foreign gene is a chimeric gene regulated by a constitutive promoter.

5. A method of Claim 1 wherein the bacteria express a foreign gene which encodes a glycosidase enzyme.

6. A method of Claim 5 wherein the cells are fluorescent pseudomonads and the glycosidase enzyme is beta-galactosidase.

7. A genetically transformed bacteria which:

a. is capable of proliferating under conditions of microbial competition, in an environment which is infested by or susceptible to nematodes; and

b. releases at least one enzyme expressed by a foreign gene which inhibits one or more types nematodes.

8. A genetically transformed bacteria of Claim 7 wherein the enzyme comprises chitinase.

9. A genetically transformed bacteria of Claim 7 wherein the enzyme comprises a glycosidase enzyme.

10. A genetically transformed bacteria of Claim 7 which releases chitinase and a glycosidase enzyme.

11. A genetically transformed bacteria of Claim 7 which expresses a chimeric foreign gene which is regulated by constitutive promoter.

12. A genetically transformed bacteria of Claim 7 wherein the environment comprises the root surfaces of one or more types of plant.

13. A genetically transformed bacteria of Claim 12 wherein the enzyme comprises chitinase

14. A genetically transformed bacteria of Claim 12 wherein the enzyme comprises a glycosidase enzyme.

15. A genetically transformed bacteria of Claim 12 which belongs to the family of Pseudomonadaceae.

16. A genetically transformed bacteria of Claim 12 which belongs to a genus selected from the group consisting of Agrobacterium, Rhizobium, Azotobacter, Azospirrillum, Flavobacterium, Acetobacter, Erwinia, Enterobacter, Klebsiella, Serratia, Citrobacter, Chromobacterium, Proteus, Gluconobacter, Corynebacterium, Streptomyces, Frankia, Nocardia, and Bacillus.

17. A genetically transformed bacteria of Claim 12 wherein the foreign gene comprises a chimeric gene which is regulated by a constitutive promoter.

18.  A fluorescent pseudomonad which expresses at least one foreign gene, which colonizes the root surfaces of one or more types of plants, and which inhibits nematodes, wherein the foreign gene is regulated by a constitutive promoter, and wherein the foreign gene encodes an enzyme selected from the group consisting of chitinase and glycosidase.

19.  A fluorescent pseudomonad of Claim 18 selected from the group consisting of strains 701L5 (ATCC No. 53175) and 1141L1 (ATCC No. 53170).

20.  Plant tissue which has been inoculated with a bacteria of Claim 7.

21. Plant tissue which has been inoculated with a bacteria of Claim 12

22. Plant tissue which has been inoculated with a bacteria of Claim 18.

23. A plasmid selected from the group consisting of pMON5031, pMON5036, and pMON5040, and mutants and variants thereof.

24. Bacteria having an ATCC accession number selected from the group consisting of 39764, 39765, 39774, 53175, and 53176, and mutants and variants thereof.

25. Plant tissue which has been inoculated with bacteria of Claim 24.

26. Bacteria containing one or more genes descended from a culture of cells having an ATCC accession number selected from the group consisting of 39764, 39765, 39774, 53175, and 53176.

27. Plant tissue which has been inoculated with bacteria of Claim 26.

28. A method of inhibiting nematodes comprising inoculating an environment which is infested with or susceptible to nematodes with genetically transformed bacteria of Claim 7.

29. A method of inhibiting nematodes comprising inoculating an environment where nematodes occur with genetically transformed bacteria of Claim 12.

30. A method of inhibiting nematodes comprising inoculating an environment where nematodes occur with a genetically transformed bacteria of Claim 18.

31. A composition of matter for inoculating an environment that is infested with or susceptible to nematodes, comprising a selected carrier material, and bacteria of Claim 1.

32. A composition of matter for inoculating an environment that is infested with or susceptible to nematodes, comprising a selected carrier material, and genetically transformed bacteria of Claim 10

33. A composition of matter of Claim 32, wherein the carrier material comprises material that is selected from the group consisting of peat moss, soil, sand, vermiculite, plant tissue, methylcellulose, and xanthan gum.

34. A composition of matter of Claim 32, wherein the bacteria are treated to prolong their viability or decrease their levels of metabolic activity during handling.

35. A composition of matter of Claim 32, wherein the carrier material contains nutrient sources that are assimilable by the bacteria.

36. A composition of matter of Claim 32, wherein the bacteria are descended from a culture of cells having an ATCC accession number selected from the group consisting of 39764, 39765, 39774, 53175, and 53176.

0171381

1/5

FIG. I.

FIG. 2.

FIG. 3.

ULTROGEL AcA 54 CHROMATOGRAPHY

FIG. 4.

EFFECT OF CHITINASE TREATMENT
ON THE EGG HATCH OF THE ROOT-KNOT NEMATODE

FIG. 5.